# EUROPEAN PATENT APPLICATION

(11) **EP 3 965 119 A1**
(43) Date of publication of application: **09.03.2022**
(21) Application number: 20194511.0
(22) Date of filing: 04.09.2020
(51) Int. Cl.: G16H 50/30, G16H 20/10, C12Q 1/6886

(54) **METHODS FOR ESTIMATING HETEROGENEITY OF A TUMOUR BASED ON VALUES FOR TWO OR MORE GENOME MUTATION AND/OR GENE EXPRESSION RELATED PARAMETER, AS WELL AS CORRESPONDING DEVICES**

(71) Applicant: Koninklijke Philips N.V., 5656 AG Eindhoven (NL)
(72) Inventor: ALVES DE INDA, Márcia, 5656 AE Eindhoven (NL); VAN DE STOLPE, Anja, 5656 AE Eindhoven (NL); BIKKER, Jan WIllem, 5656 AE Eindhoven (NL)
(74) Representative: Philips Intellectual Property & Standards

(57) **Abstract**

A method for estimating heterogeneity of a tumour based on values for two or more genome mutation and/or gene expression related parameters for at least two spatially differentiated areas in a sample, the sample being a tissue sample of a tumour or a liquid sample obtained from a subject having the tumour, the method comprising the steps of determining a sample heterogeneity score for the heterogeneity of the sample based on variabilities of each measured value for the at least two spatially differentiated areas, estimating the heterogeneity of the tumour by extrapolating the score for the heterogeneity of the sample to the tumour, thereby providing a tumour heterogeneity score.

## Description

### FIELD OF THE INVENTION

The present invention generally relates to tumours and, more specifically, to methods for estimating the heterogeneity of tumours.

### BACKGROUND OF THE INVENTION

To diagnose cancer and decide on the best therapy, a tumour tissue biopsy sample is taken from the tumour and analysed by the pathologist, that is, histopathology analysis is performed. Recently molecular analysis has been added to provide more information on the cause of the cancer and on cancer cell behaviour and response or resistance to the various possible therapies, including targeted drugs. Usually a single biopsy block is obtained by a biopsy from a tumour, be it a primary or a metastatic tumour that needs treatment.

Targeted drugs are a relatively novel category of drugs that target the underlying pathophysiology in the tumour. This pathophysiology can be described in terms of activity of 10-15 cellular signal transduction pathways, that can potentially drive tumour growth and metastasis, e.g. the estrogen, progesterone, and androgen receptor (respectively ER, PR, and AR) pathways, the PI3K, MAPK, STAT1/2 and STAT3 growth factor pathways, the Wnt, Hedgehog, Notch and TGFbeta developmental pathways, the inflammatory NFkB pathway. Targeted drugs have been developed to inhibit one of these pathways, e.g. tamoxifen which inhibits the ER pathway. They can be used in a neo-adjuvant setting, meaning the primary tumour is treated prior to surgical resection, as a primary therapy when surgery is not possible, or in a metastatic setting when surgical removal of the metastatic tumour(s) is not useful anymore. Additionally targeted drugs may be used as an adjuvant therapy, i.e. as a complementary therapy after surgical resection of the tumor.

However, these targeted drugs, and other targeted treatments, are only effective in the treatment of a tumour in which the targeted pathway is active and driving the tumour growth. In general, within a cancer type various signalling pathways can be the tumour-driving pathway, and only a subgroup of patients will have a tumour driven by the same pathway. This makes it very important to define the tumour driving pathway(s) in each individual cancer to treat, prior to installing treatment.

In recent years, it has become clear that a tumour is generally not homogeneous with respect to cancer cell genotype and phenotype. Clonal evolution of cancer has been theoretically described to lead to (1) a few major cancer cell clones in a tumour, separating the tumour in relatively large areas with distinctly different behaviour and, for example, response to therapy; or alternatively (2) a large number of smaller clones with similar genotype and phenotype distributed over the tumour, such that in any area the same distribution of genotypic/phenotypic clones is found, the latter is called the "Big Bang theory for cancer evolution".

The phenotype of the tumour may thus vary, or not, and a single biopsy sample analysed from a tissue block taken from a certain location in the tumour may therefore not be sufficiently representative for the whole tumour.

Currently, treatment is based on measurements done on a single (biopsy) sample and, if that single (biopsy) sample is not representative of the whole, a less optimal treatment choice may be the consequence. Optimizing targeted therapy choice requires that the analysed tissue on which the therapy decision is taken is representative for the whole tumour.

The straightforward solution to this problem would be to collect multiple tissue biopsies from different parts of the tumour. However, this is an additional burden to the patient that may induce side effects, and therefore in general is not clinically adopted.

Following the above, there is a need for determining or estimating the heterogeneity of a tumour in a manner that avoids additional burden for the patient, and thus also avoids additional side effects.

### SUMMARY OF THE INVENTION

It is an object of the present invention to provide for a friendly and effective method for estimating the heterogeneity of a tumour in a patient. Further objects of the present invention include an apparatus, a storage medium, a computer program, a signal and the use of the apparatus all associated with the presented method.

According to a first aspect, a method is provided for estimating heterogeneity of a tumour based on values of two or more genome mutation and/or gene expression related parameters for at least two spatially differentiated areas in a sample, the sample being a tissue sample of a tumour or a liquid sample obtained from a subject having the tumour, the method comprising the steps of:
- determining a sample heterogeneity score for the heterogeneity of the sample based on variabilities of each measured value for the at least two spatially differentiated areas;
- estimating the heterogeneity of the tumour by extrapolating the score for the heterogeneity of the sample to the tumour, thereby providing a tumour heterogeneity score.

The present disclosure is directed to a concept in which the heterogeneity of a tumour may be estimated based on a single sample of the tumour. The heterogeneity of the specific single sample may then be extrapolated to the whole tumour.

In an example, the present disclosure may be considered as a method to predict, based on signalling pathway analysis of a single biopsy block of a tumour, the heterogeneity within the whole tumour with respect to activity of signal transduction pathway activity.

By being able to define how heterogeneous a tumour is with the use of one single biopsy block, i.e. one single sample, no risk is added for the patient, while additional information is provided on the whole tumour heterogeneity with respect to signalling pathway activity. This is expected to provide support to the physician in deciding whether or not one or more additional biopsies are necessary to obtain a reliable picture of the signalling pathway activity across the whole tumour.

The present disclosure may also improve the process of choosing (targeted) therapy for the individual patient with cancer, may contribute to personalized medicine and may be expected to lead to more effective treatment and improve clinical outcome.

It is thus the insight of the inventors that the tissue sample taken from a (small) area of the tumour is informative, or representative, for the whole tumour. This would make obtaining additional (spatially distributed) samples from the same tumour superfluous.

More specifically, it has been found that the heterogeneity of a tumour is similar if not larger on a microscopic scale compared to macroscopic scale. This, thus, enables to define, or estimate, a heterogeneity score representative of the whole tumour based on a single biopsy sample by sub-sampling the biopsy sample and estimating the heterogeneity score based on the variation of the molecular composition of the sub-samples.

In an example, the step of determining the sample heterogeneity score comprises the step of performing a Principal Component Analysis, PCA, for converting the two or more genome mutation and/or gene expression related parameters into principle components, thereby reducing correlations between the two or more genome mutation and/or gene expression related parameters.

Many different genome mutation and/or gene expression related parameters exist in a tumour. Unfortunately, these parameters may be correlated with each other such that it is more difficult to assess which parameter contributes the most to the heterogeneity of a tumour, and how much of the heterogeneity of tumour is caused by a particular parameter or set of parameters.

The inventors have found at least two methods to tackle the above.

The first method does not correct for the above mentioned correlations. For example, if it is known which parameters are most important, or vary the most, in a tumour, we could simply focus the presented method on these particular parameters. The influence of other genome mutation and/or gene expression related parameters is then ignored. The end result will therefore have the most important factors required by a physician for, for example, determining a therapy.

Following the above first described method, a physician may be informed that a particular influential parameter is varying or not. Even such an insight could be helpful for the physician in determining the correct therapy.

In an example, if the ER pathway is found active in the sample and analyses of the sample reveals that the tumor is homogenous (e.g. by using signalling pathway activities or other genome mutation and/or gene expression related parameters), one could conclude that the ER pathway is homogenously active in the tumor and this is the pathway driving the tumour growth. A single targeted drug can be prescribed. If, on the other hand, the tumour is heterogeneous, a systemic treatment (such as chemotherapy) or a second targeted drug could be considered.

It is further noted that, in the first method, the mean values for each of the two or more genome mutation and/or gene expression related parameters based on the measured values may be determined, and that the each of the measured values may be subtracted by a mean value of its corresponding genome mutation and/or gene expression related parameter. The end result may then be a measure for the heterogeneity of the tumour which a physician can use for determining the appropriate treatment. The second method does correct for the above mentioned correlations. This may be accomplished by performing a Principal Component Analysis. PCA.

Here, the PCA is performed in such a way that it uses an orthogonal transformation to convert a two or more genome mutation and/or gene expression related parameters, being possibly correlated variables, into a set of values of linearly uncorrelated variables which are called principal components.

By performing a PCA, the base of the space, which could be compared to "selecting the best camera position", is transformed such that in the new basis the first component will have the largest possible variance (this will be the most important direction), the second component will have the second largest variance, etc. As a consequence the correlations will be reduced.

By performing the PCA, a more reliable, or accurate, estimation may be made on the heterogeneity, as the covariance, i.e. the measure of the joined variability of the different genome mutation and/or gene expression related parameters, is reduced. This, thus, provides for a more accurate estimation of the heterogeneity of the tumour.

In an example hereof, the step of performing the PCA comprises:
- determining mean values for each of the two or more genome mutation and/or gene expression related parameters based on the measured values;
- subtracting each of the measured values by a mean value of its corresponding genome mutation and/or gene expression related parameter.

The PCA may, basically, comprise a plurality of steps. First, the data set is organized and a plurality of parameters are chosen. Mean values for each of the chosen parameters are determined based on their measured appearance in the sample. Then, the covariance matrix may be determined, and the eigenvector and eigenvalues of the covariance matrix may be determined. The eigenvectors and eigenvalues are then rearranged for reducing the covariance.

This may also be explained as follows. First, the number of parameters are reduced to obtain a reduced number of parameters to monitor, i.e. signalling pathway activities, percentage of important mutations, expression of key genes, etc. So, the total amount of parameters may be reduced by concentrating on a few actionable ones, i.e. from maybe thousands of gene expression values where each gene may be considered as important as the other to a few of well-defined cellular signalling pathway activities that may be used to facilitate diagnosis or therapy selection.

Then, in a preferential example/embodiment, a frozen base is created in which to transform the reduced number of parameters. That is, to be able to estimate the heterogeneity of a single tumour without having to directly compare with other tumours, first a basis may be defined in which it is possible to project any sample separately. The method may thus also comprise the step of providing a base transformation matrix, M, constructed from samples from different areas of tumours of multiple subjects.

Finally, the heterogeneity score is determined by transforming the parameters of multiple subsamples of a single sample of a patient into the space created by the frozen base as a way to represent the molecular constitution of each subsample by a multi-dimensional vector. The resulting multi-dimensional vectors may then be combined by first estimating the variability in each direction and then summarizing the multi-dimensional variability vectors into one single score.

Following the above, in a further example, the step of determining the sample heterogeneity score comprises the steps of:
- computing standard deviations for each of the principle components and determining the sample heterogeneity score for the heterogeneity of the sample based on the computed standard deviations.

In a further example, the method further comprises one or more steps selected from:
- deciding a treatment strategy for the subject based at least in part on the tumour heterogeneity score;
- predicting a treatment outcome for the subject based at least in part on the tumour heterogeneity score;
- predicting a survival probability for the subject based at least in part on the tumour heterogeneity score;
- predicting resistance to a therapy based at least in part on the tumour heterogeneity score;
- deciding on the efficacy of therapy administered prior to the heterogeneity analysis;
- deciding on resistance to therapy administered prior to the heterogeneity analysis.

It was found that the presented method of estimating the heterogeneity of a tumour may be used in several cases. The above described options provide for a non-exhausted list of potential candidates. Other uses of the estimated heterogeneity score are also encompassed by the present disclosure.

In accordance with the present disclosure, at least two spatially differentiated areas in a (biopsy) sample are used in which values for two or more genome mutation and/or gene expression related parameters are measured. In a specific embodiment, four quadrants of a single biopsy of the tumour of a particular patient may be used. This would lead to four values for each parameter, thereby increasing the accuracy of the process of estimating the heterogeneity.

In an example, the gene expression related parameters are gene expression levels of three or more target genes each of one or more cellular signalling pathway selected from the group consisting of ER, AR, HH, PI3K-FOXO, WNT, TGFbeta, NFkB, JAK-STAT1/2, JAK-STAT3, Notch, PR and MAPK-AP1, preferably wherein said gene expression related parameters are cellular signalling pathway activities based on the three or more target genes expression levels for said cellular signalling pathway, more preferably wherein said cellular signalling pathway activity is selected from the group consisting of ER, AR, HH, PI3K-FOXO, WNT, TGFbeta, NFkB, JAK-STAT1/2, JAK-STAT3, Notch, PR and MAPK-AP1 cellular signalling pathway activity.

The inventors have found that a particularly suitable genome mutation and/or gene expression related parameters are the expression levels of three or more, target genes of cellular signaling pathways. The expression levels of these target genes can be used to determine the cellular signaling pathway activity using a mathematical model as described below. Therefore the expression levels of the target genes of the cellular signaling pathways may be used directly in the method as disclosed herein, or the expression levels of the target genes can used to determine the activity or activities of one or more cellular signaling pathways, and the method disclosed herein can be based on the determined cellular signaling activity or activities. Preferably the cellular signaling pathway activity is one or more selected from the group consisting of ER, AR, HH, PI3K-FOXO, WNT, TGFbeta, NFkB, JAK-STAT1/2, JAK-STAT3, Notch, PR and MAPK-AP1. By using e.g. a mathematical model the cellular signalling pathway activity can be represented by a numerical value, this numerical value representing the cellular signalling pathway activity can be applied as a directly actionable parameter in the methods described herein. It was found that differences in cellular signalling pathway activity are particularly useful in determining heterogeneity in a tumour as the pathway activities display a large degree of variation among different cell types (or subtypes) while displaying minimal variation between similar or identical cell types (or subtypes), unlike for example the genetic variation within a tumour or the gene expression patterns within a tumour.

In an example, the step of estimating said sample heterogeneity score for the heterogeneity of the sample, comprises any of:
- multiplying each of said variabilities of each measured value for the at least two spatially differentiated areas, and
- summing each of said variabilities of each measured value for the at least two spatially differentiated areas.

The inventors have found that the heterogeneity score may, ultimately, be determined in several manners. Each of the variabilities of each measured value for the at least two spatially differentiated areas may be multiplied, summed, or may be tackled in any other manner.

In yet another example, the step of estimating the heterogeneity of the tumour comprises:
- setting an upper bound for the tumour heterogeneity score as the sample heterogeneity score.

It was found that the heterogeneity score of a sub-sample may be extrapolated to the heterogeneity score for the whole tumour. However, it was further found that it is likely that, at least for some features, the heterogeneity at sub-sample level is higher (or similar) compared to the heterogeneity of the corresponding tumour. As such, by extrapolating the results for the sub-sample level, an upper-bound may be set for the heterogeneity of the whole tumour. This indicates to the physician that the heterogeneity of the whole tumour is, most likely, at most equal to the heterogeneity at the sub-sample level.

In a second aspect of the present disclosure, an apparatus is provided comprising a processor configured to perform a method in accordance with any of the examples provided above.

The apparatus may, for example, be responsible for
- scanning said sample
- identifying, in said sample, an area of interest;
- measuring, for said area of interest, value for the at least two spatially differentiated areas for two or more genome mutation and/or gene expression related parameters.

It is noted that the apparatus in accordance with the present disclosure may thus be arranged to scan the sample using a camera or the like. An area of interest may be detected in the scanned sample, for example an area related to the tumour. The apparatus may further comprise cellomatic means, i.e. as a standalone entity or integrated in the apparatus itself, for marking the tumour area in the scanned area and for extracting it. Finally, computing means, in combination with the processor, may be provided for performing, amongst other, the steps of determining a sample heterogeneity score for the heterogeneity of the sample based on variabilities of each measured value for the at least two spatially differentiated areas, and for estimating the heterogeneity of the tumour by extrapolating the score for the heterogeneity of the sample to the tumour, thereby providing a tumour heterogeneity score.

In a further aspect, a non transitory storage medium is provided storing instructions that are executable by a processor to perform a method in accordance with any one of the examples as provided above.

In yet another aspect, a computer program is provided comprising program code means for causing a processor to perform a method in accordance with any of the examples as provided above.

In an even further aspect, there is provided a signal representing a tumour heterogeneity score that indicates the heterogeneity of a tumour, wherein the tumour heterogeneity score results from performing a method in accordance with any of the examples as provided above.

In a final aspect, there is provided use of the apparatus, the non-transitory storage medium, the computer program and/or the signal as disclosed above for diagnosing the subject, predicting a treatment outcome for the subject or predicting an optimal treatment strategy for the subject, wherein the diagnosing or predicting is based on the tumour heterogeneity score.

### BRIEF DESCRIPTION OF THE DRAWINGS

Fig 1 discloses a tumour having multiple quadrants, wherein a single quadrant is used for estimating heterogeneity in accordance with the present disclosure;
Fig. 2 discloses variances of ER pathways in different types of breast cancer types, wherein the variance is either between quadrants, or within a single quadrant;
Fig. 3 discloses, visually, the use of a Principal Component Analysis, PCA, in accordance with the present disclosure;
Fig. 4 discloses an example of an apparatus in accordance with the present disclosure.

### DETAILED DESCRIPTION OF EMBODIMENTS

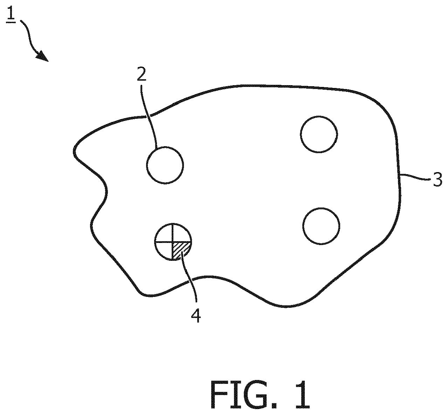

Fig 1 discloses a tumour 1 having multiple quadrants 2, 3, 4, wherein a single quadrant 4 is used for estimating heterogeneity in accordance with the present disclosure.

The text below is referenced to signalling pathway activities. However, it may be noted that the present disclosure is applicable for estimating heterogeneity of a tumour based on values for any two or more genome mutation and/or gene expression related parameters.

The present disclosure is, in an example, directed to a method to decide on targeted drug treatment based on a heterogeneity score of a tumour, for example with respect to signalling pathway activity. As such, in a first step, it is assumed that a particular tumour may be heterogeneous, and the degree of heterogeneity of the tumour is a factor for deciding the applicable drug treatment.

In a second step, it is assumed that the degree of heterogeneity of a single biopsy, i.e. for example a single quadrant as shown in Fig. 1, is representative for the whole tumour. This does not necessarily mean that the heterogeneity in a subsample of the tumour equals the heterogeneity of the whole tumour. The above means that the degree of heterogeneity in the subsample is a measure for the degree of heterogeneity of the whole tumour. More specifically, in an example, it was found that it is likely that the degree of heterogeneity in a subsample of the tumour is equal or higher compared to the degree of heterogeneity of the whole tumour, thereby setting an upper bound of the degree of heterogeneity of the whole tumour.

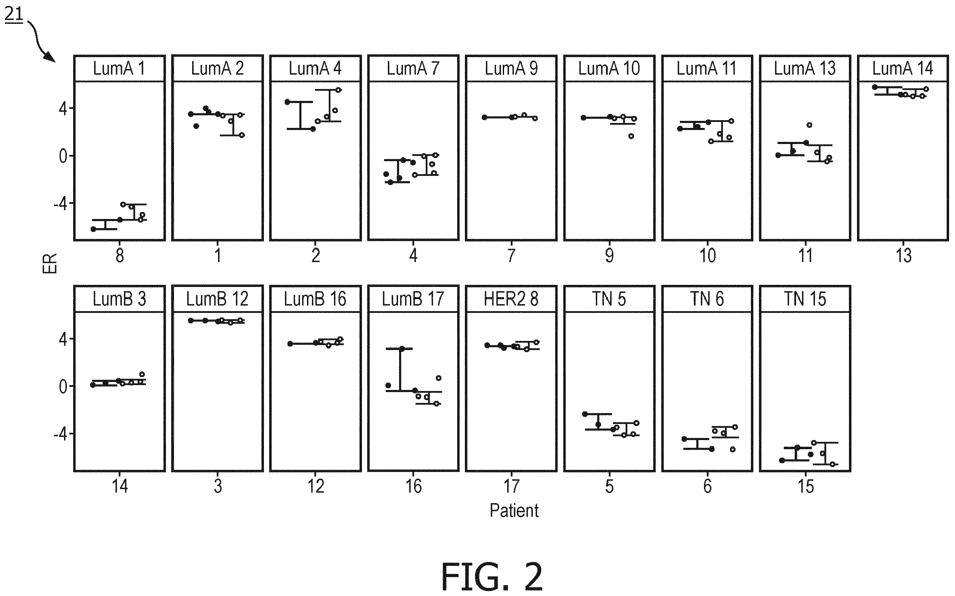

One of the pathways that may be examined is the ER pathway in different types of breast cancers, which is shown in Fig. 2.

Here, the different types 21 of breast cancer are indicated with the wording 'LumA 1", "LumA 2", "LumA 4", ....., "TN 5, "TN6", "TN 15".

The vertical scale indicates the value with respect to the ER pathway for the given type of breast cancer.

Each graph comprises two sets of data points. The left part of each graph is related to data points between quadrants, the right part of each graph is directed to data point within a quadrant, i.e. subsample.

For example, for LumA 1, the value of the ER pathway between quadrants, i.e. for the whole tumour, ranges from about -6 to about -5. The ER pathway within a quadrant, i.e. subsample, ranges from about -5 to about -4. From the graphs shown in Fig. 2, it may be deduced that the vertical range between data points is, in general, comparable. That is, the variance of the ER pathway within a subsample may be representative for the variance of the ER pathway within the whole tumour.

The inventors have found that the heterogeneity of the whole tumour may be estimated in at least two different methods.

It is noted that it may be difficult to estimate the heterogeneity of a single parameter, as the parameters may all be correlated with each other. That is parameters may have a joint variability between them. It may therefore be difficult to assess which of the parameters is the dominant one, i.e. the one that is dominant for the heterogeneity of the tumour. The first method does not correct for the above described joint variability between the parameters. The second method does correct for the joint variability by introducing a PCA.

Let's consider the first method. Here, a sample heterogeneity score for the heterogeneity of the sample may be determined based on variabilities of each measured value for the at least two spatially differentiated areas. So, a single sample, being for example a tissue sample or a liquid sample, may be divided into multiple subsamples.

The values for two or more genome mutation and/or gene expression related parameters may then be measured in each of the subsamples. The deltas of the values for a parameter between different subsamples may then be used to estimate, or determine, the heterogeneity of the sample. The heterogeneity of the sample may then be extrapolated to the whole tumour.

The above described deltas may be the range between the min-max values between measured pathway activities in the subsamples.

The above described method may be visualized using Fig. 2. Here, the range, i.e. the vertical distance between the measurement points of a parameters within a particular sample is used for determining the heterogeneity of the whole tumour. Let's consider the LumAl situation. On the left side it is shown that the range between the measurement points, i.e. measuring the ER pathway, is approximately equal to 1, i.e. ranging from about -6 to about -5. This indicates a particular heterogeneity in the sample. This value may then be extrapolated, or may be simply used, as a measure for the whole tumour. This is shown on the right side of the same figure. Here, the actual measurement point may differ from the left side, but the range between the measurement points seem to be equal, or at least comparable.

The second method does correct for the above mentioned correlations. This may be accomplished by performing a Principal Component Analysis. PCA.

Here, the PCA may be performed in such a way that it uses an orthogonal transformation to convert two or more genome mutation and/or gene expression related parameters, being possibly correlated variables, into a set of values of linearly uncorrelated variables which are called principal components.

Let's consider a situation in which the ER, AR, WNT, FOXO, TGFbeta and HH pathways are taken into account. This may thus be a reduced set of parameters. All these parameters may have a correlation with each other. That is, a covariance matrix may exist, which is a square matrix giving the covariance between each pair of parameters. In the diagonal of such a matrix diagonal there are variances, i.e., the covariance of each element with itself.

The first described method thus looks at the variance of a single parameter without correcting for the covariance caused by other parameters.

The second uses a principal component analysis. The basic idea of a principal component analysis is to reduce the dimensionality of a data set, which data set comprises multiple parameters / variables that are correlated with each other, while retaining the variation present in the data set as much as possible. This is done by transforming the parameters / variables to a new set of variables which are called principal component. These principal components are orthogonal and ordered such that the retention of variation present in the original variables / parameters decreases as we move down the order. So, in this way, the 1^{st} principal component retains the most variation that was present in the original components. In mathematic terms, the principal components are the eigenvectors of the covariance matrix, and they are therefore orthogonal.

Given the above, using the PCA method, the, for example, ER, AR, WNT, FOXO, TGFbeta and HH pathways are transformed into principal components and are, subsequently, ordered. This thus indicated which of these variables have the highest variance itself, and is therefore thus also the dominant factor in the heterogeneity of the whole tumour.

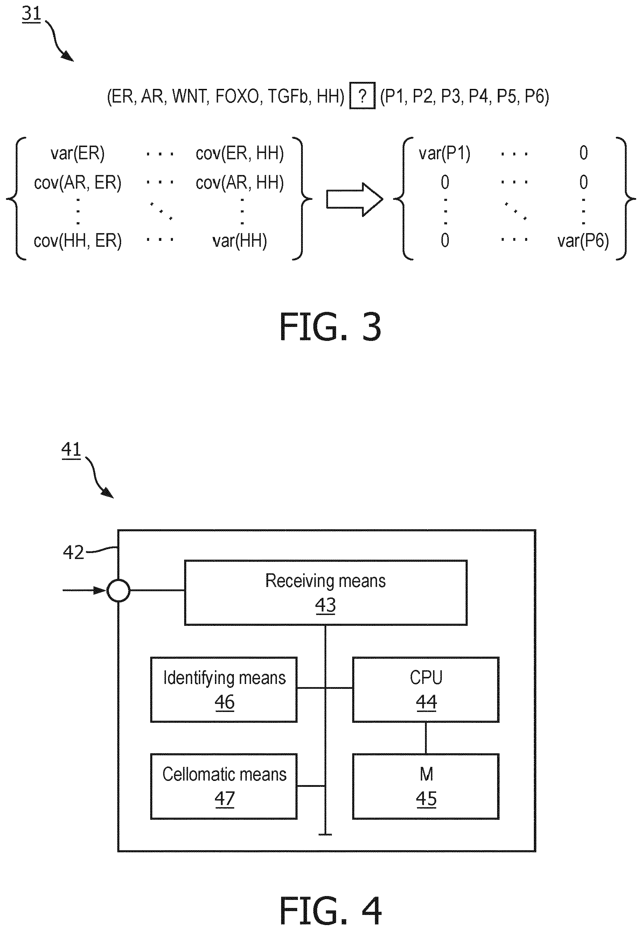

The PCA method 3 is indicated, visually, in Fig. 3, wherein the transformation of the covariance matrix to the principal components is shown.

Although the above examples demonstrate the use of the cellular signalling pathway activities for the ER, AR, WNT, FOXO, TGFbeta and HH pathways, it will be evident to the skilled person that other cellular signalling pathways, or genome mutation and/or gene expression related parameters, may be used in the methods disclosed herein.

Cellular signalling pathway activities can be determined based on the expression levels of three or more, target genes for each respective cellular signalling pathway using a mathematical model. By using a calibrated mathematical model to relate the target gene expression levels to a cellular signalling pathway activity, a numerical value can be assigned to the pathway activity. Depending on the model, this value can for example be normalized to result in a value from 0 to 100, where 0 is no pathway activity and 100 is the theoretical maximum pathway activity. Alternatively the value may be normalized such that the average value is 0 and thus decreased pathway activity is represented by a negative value and increased pathway activity is represented by a positive value. It is understood that the values obtained using such model are dependent on the model used, and do not represent absolute values. Therefore, the same model should be used for calibrating, determining reference values and when used in the method of the invention, so that it allows comparison of the obtained numerical values for pathway activity.

Fig. 4 discloses an example of an apparatus 41 in accordance with the present disclosure.

The apparatus comprises a processor 44 in communication with memory 45.

Data 42 may be received from, for example, a scanner or the like. The data may resemble the image taken from a particular sample. The receiving means 43 is responsible for receiving the data.

Identifying means 46 may be present for marking an area in the image resembled by the data. The marked area may be representative for a tumour. cellomatic means 47 may be provided for marking the tumour area in the scanned area and for extracting it. Finally, the processing of the data, i.e. for obtaining the heterogeneity score, may be performed by the CPU 44.

The terms "pathway", "signal transduction pathway", "signalling pathway" and "cellular signalling pathway" are used interchangeably herein.

An "activity of a signalling pathway" may refer to the activity of a signalling pathway associated transcription factor (TF) element in the sample, the TF element controlling transcription of target genes, in driving the target genes to expression, i.e., the speed by which the target genes are transcribed, e.g. in terms of high activity (i.e. high speed) or low activity (i.e. low speed), or other dimensions, such as levels, values or the like related to such activity (e.g. speed). Accordingly, for the purposes of the present invention, the term "activity", as used herein, is also meant to refer to an activity level that may be obtained as an intermediate result during "pathway analysis" as described herein.

The term "transcription factor element" (TF element), as used herein, preferably refers to an intermediate or precursor protein or protein complex of the active transcription factor, or an active transcription factor protein or protein complex which controls the specified target gene expression. For example, the protein complex may contain at least the intracellular domain of one of the respective signalling pathway proteins, with one or more co-factors, thereby controlling transcription of target genes. Preferably, the term refers to either a protein or protein complex transcriptional factor triggered by the cleavage of one of the respective signalling pathway proteins resulting in a intracellular domain.

The calibrated mathematical pathway model is preferably a centroid or a linear model, or a Bayesian network model based on conditional probabilities. For example, the calibrated mathematical pathway model may be a probabilistic model, preferably a Bayesian network model, based on conditional probabilities relating the target gene expression levels and the activities of the signalling pathways, or the calibrated mathematical pathway model may be based on one or more linear combination(s) of the expression levels of target genes of the signalling pathways.

According to a preferred embodiment of the present invention, the activity of the respective signal pathway is determined or determinable by pathway analysis as described herein.

Pathway analysis enables quantitative measurement of signal transduction pathway activity in epithelial cells, based on inferring activity of a signal transduction pathway from measurements of mRNA levels of the well-validated direct target genes of the transcription factor associated with the respective signalling pathway (see for example W Verhaegh et al., 2014, supra; W Verhaegh, A van de Stolpe, Oncotarget, 2014, 5(14):5196).

Preferably the determining of the activities of the signalling pathways, the combination of multiple pathway activities and applications thereof is performed as described for example in the following documents, each of which is hereby incorporated in its entirety for the purposes of determining activity of the respective signalling pathway: published international patent applications WO2013011479 (titled "ASSESSMENT OF CELLULAR SIGNALING PATHWAY ACTIVITY USING PROBABILISTIC MODELING OF TARGET GENE EXPRESSION"), WO2014102668 (titled "ASSESSMENT OF CELLULAR SIGNALING PATHWAY ACTIVITY USING LINEAR COMBINATION(S) OF TARGET GENE EXPRESSIONS"), WO2015101635 (titled "ASSESSMENT OF THE PI3K CELLULAR SIGNALING PATHWAY ACTIVITY USING MATHEMATICAL MODELLING OF TARGET GENE EXPRESSION"), WO2016062891 (titled "ASSESSMENT OF TGF-β CELLULAR SIGNALING PATHWAY ACTIVITY USING MATHEMATICAL MODELLING OF TARGET GENE EXPRESSION"), WO2017029215 (titled "ASSESSMENT OF NFKB CELLULAR SIGNALING PATHWAY ACTIVITY USING MATHEMATICAL MODELLING OF TARGET GENE EXPRESSION"), WO2014174003 (titled "MEDICAL PROGNOSIS AND PREDICTION OF TREATMENT RESPONSE USING MULTIPLE CELLULAR SIGNALLING PATHWAY ACTIVITIES"), WO2016062892 (titled "MEDICAL PROGNOSIS AND PREDICTION OF TREATMENT RESPONSE USING MULTIPLE CELLULAR SIGNALING PATHWAY ACTIVITIES"), WO2016062893 (titled "MEDICAL PROGNOSIS AND PREDICTION OF TREATMENT RESPONSE USING MULTIPLE CELLULAR SIGNALING PATHWAY ACTIVITIES"), WO2018096076 (titled "Method to distinguish tumour suppressive FOXO activity from oxidative stress"), and in the patent applications EP16200697.7 (filed on Nov 25, 2016; titled "Method to distinguish tumour suppressive FOXO activity from oxidative stress"), EP17194288.1 (filed on Oct 2, 2017; titled "Assessment of Notch cellular signalling pathway activity using mathematical modelling of target gene expression"), EP17194291.5 (filed on Oct 2, 2017; titled "Assessment of JAK-STAT1/2 cellular signalling pathway activity using mathematical modelling of target gene expression"), EP17194293.1 (filed on Oct 2, 2017; titled "Assessment of JAK-STAT3 cellular signalling pathway activity using mathematical modelling of target gene expression") and EP17209053.2 (filed on Dec 20, 2017, titled "Assessment of MAPK-AP1 cellular signalling pathway activity using mathematical modelling of target gene expression"), PCT/EP2018/076232 (filed on Sep 27, 2018, titled "Assessment of JAK-STAT3 cellular signalling pathway activity using mathematical modelling of target gene expression"), PCT/EP2018/076334 (filed on Sep 27, 2018, titled "Assessment of JAK-STAT1/2 cellular signalling pathway activity using mathematical modelling of target gene expression"), PCT/EP2018/076488 (filed on Sep 28, 2018, titled "Assessment of Notch cellular signalling pathway activity using mathematical modelling of target gene expression"), PCT/EP2018/076513 (filed on Sep 28, 2018, titled "Assessment of MAPK-AP-1 cellular signalling pathway activity using mathematical modelling of target gene expression"), and PCT/EP2018/076614 (filed on Oct 1, 2018, titled "Determining functional status of immune cells types and immune response").

The models have been biologically validated for ER, AR, PI3K-FOXO, HH, Notch, TGF-β, Wnt, NFkB, JAK-STAT1/2, JAK-STAT3 and MAPK-AP1 pathways on several cell types.

Unique sets of cellular signalling pathway target genes whose expression levels are preferably analyzed have been identified. For use in the mathematical models, three or more, for example, three, four, five, six, seven, eight, nine, ten, eleven, twelve or more, target genes from each assessed cellular signalling pathway can be analyzed to determine pathway activities.

Common to the pathway analysis methods for determining the activities of the different signalling pathways as disclosed herein is a concept, which is preferably applied herein for the purposes of the present invention, wherein the activity of a signalling pathway in a cell such as an epithelial cell present in a sample is determinable by receiving expression levels of three or more, target genes of the signalling pathway, determining an activity level of a signalling pathway associated transcription factor (TF) element in the sample, the TF element controlling transcription of the three or more target genes, the determining being based on evaluating a calibrated mathematical pathway model relating expression levels of the three or more target genes to the activity level of the signalling pathway, and optionally inferring the activity of the signalling pathway in the epithelial cell based on the determined activity level of the signalling pathway associated TF element. As described herein, the activity level can be directly used as an input in the method disclosed herein, for example as a principle component in the principle component analysis.

The term "activity level" of a TF element, as used herein, denotes the level of activity of the TF element regarding transcription of its target genes.

The calibrated mathematical pathway model may be a probabilistic model, preferably a Bayesian network model, based on conditional probabilities relating the activity level of the signalling pathway associated TF element and the expression levels of the three or more target genes, or the calibrated mathematical pathway model may be based on one or more linear combination(s) of the expression levels of the three or more target genes. For the purposes of the present invention, the calibrated mathematical pathway model is preferably a centroid or a linear model, or a Bayesian network model based on conditional probabilities.

In particular, the determination of the expression level and optionally the inferring of the activity of a signalling pathway in the subject may be performed, for example, by inter alia (i) evaluating a portion of a calibrated probabilistic pathway model, preferably a Bayesian network, representing the cellular signalling pathways for a set of inputs including the expression levels of the three or more target genes of the cellular signalling pathway measured in a sample of the subject, (ii) estimating an activity level in the subject of a signalling pathway associated transcription factor (TF) element, the signalling pathway associated TF element controlling transcription of the three or more target genes of the cellular signalling pathway, the estimating being based on conditional probabilities relating the activity level of the signalling pathway associated TF element and the expression levels of the three or more target genes of the cellular signalling pathway measured in the sample of the subject, and optionally (iii) inferring the activity of the cellular signalling pathway based on the estimated activity level of the signalling pathway associated TF element in the sample of the subject. This is described in detail in the published international patent application WO 2013/011479 A2 ("Assessment of cellular signalling pathway activity using probabilistic modelling of target gene expression"), the contents of which are herewith incorporated in their entirety.

In an exemplary alternative, the determination of the expression level and optionally the inferring of the activity of a cellular signalling pathway in the subject may be performed by inter alia (i) determining an activity level of a signalling pathway associated transcription factor (TF) element in the sample of the subject, the signalling pathway associated TF element controlling transcription of the three or more target genes of the cellular signalling pathway, the determining being based on evaluating a calibrated mathematical pathway model relating expression levels of the three or more target genes of the cellular signalling pathway to the activity level of the signalling pathway associated TF element, the mathematical pathway model being based on one or more linear combination(s) of expression levels of the three or more target genes, and optionally (ii) inferring the activity of the cellular signalling pathway in the subject based on the determined activity level of the signalling pathway associated TF element in the sample of the subject. This is described in detail in the published international patent application WO 2014/102668 A2 ("Assessment of cellular signalling pathway activity using linear combination(s) of target gene expressions").

Further details regarding the inferring of cellular signalling pathway activity using mathematical modelling of target gene expression can be found in W Verhaegh et al., 2014, supra.

In an embodiment the signalling pathway measurements are performed using qPCR, multiple qPCR, multiplexed qPCR, ddPCR, RNAseq, RNA expression array or mass spectrometry. For example, a gene expression microarray data, e.g. Affymetrix microarray, or RNA sequencing methods, like an Illumina sequencer, can be used.

Particularly preferred is a method wherein the inferring comprises:
inferring activity of a Wnt cellular signaling pathway in the sample based at least on expression levels of three or more, e.g. three, four five six seven, eight, nine, ten eleven, twelve or thirteen or more, target gene(s) of the Wnt pathway measured in the sample selected from the group comprising or consisting of: KIAA1199, AXIN2, RNF43, TBX3, TDGF1, SOX9, ASCL2, IL8, SP5, ZNRF3, KLF6, CCND1, DEFA6 and FZD7, optionally the inferring is further based on expression levels of at least one target gene, e.g. one, two three, four, five six, seven, eight, nine, ten or more target gene(s), of the Wnt pathway measured in the sample selected from the group comprising or consisting of: NKD1, OAT, FAT1, LEF1, GLUL, REG1B, TCF7L2, COL18A1, BMP7, SLC1A2, ADRA2C, PPARG, DKK1, HNF1A and LECT2;
inferring activity of a ER cellular signaling pathway in the sample based at least on expression levels of three or more, e.g. three, four five six seven, eight, nine, ten eleven, twelve or thirteen, target gene(s) of the ER pathway measured in the sample selected from the group comprising or consisting of: CDH26, SGK3, PGR, GREB1, CA12, XBP1, CELSR2, WISP2, DSCAM, ERBB2, CTSD, TFF1 and NRIP1, optionally the inferring is further based on expression levels of at least one target gene, e.g. one, two, three, four, five, six, seven, eight, nine, ten eleven twelve or more target gene(s), of the ER pathway measured in the sample selected from the group comprising or consisting of: AP1B1, ATP5J, COL18A1, COX7A2L, EBAG9, ESR1, HSPB1, IGFBP4, KRT19, MYC, NDUFV3, PISD, PRDM15, PTMA, RARA, SOD1 and TRIM25;
inferring activity of a HH cellular signaling pathway in the sample based at least on expression levels of three or more, e.g. three, four five six seven, eight, nine, ten eleven, twelve or thirteen, target gene(s) of the HH pathway measured in the sample selected from the group comprising or consisting of: GLI1, PTCH1, PTCH2, IGFBP6, SPP1, CCND2, FST, FOXL1, CFLAR, TSC22D1, RAB34, S100A9, S100A7, MYCN, FOXM1, GLI3, TCEA2, FYN and CTSL1, optionally the inferring is further based on expression levels of at least one target gene, e.g. one, two, three, four, five, six, seven, eight, nine, ten eleven twelve or more target gene(s), of the HH pathway measured in the sample selected from the group comprising or consisting of: BCL2, FOXA2, FOXF1, H19, HHIP, IL1R2, JAG2, JUP, MIF, MYLK, NKX2.2, NKX2.8, PITRM1 and TOM1;
inferring activity of a AR cellular signaling pathway in the sample based at least on expression levels of three or more, e.g. three, four five six seven, eight, nine, ten eleven, twelve or thirteen, target gene(s) of the AR pathway measured in the sample selected from the group comprising or consisting of: KLK2, PMEPA1, TMPRSS2, NKX3_1, ABCC4, KLK3, FKBP5, ELL2, UGT2B15, DHCR24, PPAP2A, NDRG1, LRIG1, CREB3L4, LCP1, GUCY1A3, AR and EAF2, optionally the inferring is further based on expression levels of at least one target gene, e.g. one, two, three, four, five, six, seven, eight, nine, ten eleven twelve or more target gene(s), of the AR pathway measured in the sample selected from the group comprising or consisting of: APP, NTS, PLAU, CDKN1A, DRG1, FGF8, IGF1, PRKACB, PTPN1, SGK1 and TACC2;
inferring activity of a PI3K cellular signaling pathway in the sample based at least on expression levels of three or more, e.g. three, four five six seven, eight, nine, ten eleven, twelve or thirteen, target gene(s) of the PI3K pathway measured in the sample selected from the group comprising or consisting of: AGRP, BCL2L11, BCL6, BNIP3, BTG1, CAT, CAV1, CCND1, CCND2, CCNG2, CDK 1A, CDK 1B, ESR1, FASLG, FBX032, GADD45A, INSR, MXI1, NOS3, PCK1, POMC, PPARGCIA, PRDX3, RBL2, SOD2 and TNFSF10, optionally the inferring is further based on expression levels of at least one target gene, e.g. one, two, three, four, five, six, seven, eight, nine, ten eleven twelve or more target gene(s), of the PI3K pathway measured in the sample selected from the group comprising or consisting of: ATP8A1, C10orf10, CBLB, DDB1, DYRK2, ERBB3, EREG, EXT1, FGFR2, IGF1R, IGFBP1, IGFBP3, LGMN, PPM ID, SEMA3C, SEPP1, SESN1, SLC5A3, SMAD4 and TLE4, optionally the inferring is further based on expression levels of at least one target gene, e.g. one, two, three, four, five, six, seven, eight, nine, ten eleven twelve or more target gene(s), of the PI3K pathway measured in the sample selected from the group comprising or consisting of: ATG14, BIRC5, IGFBP1, KLF2, KLF4, MYOD1, PDK4, RAG1, RAG2, SESN1, SIRT1, STK11 and TXNIP, preferably inferring the activity of the FOXO/PI3K cellular signaling pathway in the sample is based at least on expression levels of at least three, target gene(s) of the FOXO/PI3K cellular signaling pathway measured in the extracted sample of the medical subject selected from the group consisting of: AGRP, BCL2L11, BCL6, BNTP3, BTG1, CAT, CAV1, CCND1, CCND2, CCNG2, CDKN1A, CDKN1B, ESR1, FASLG, FBX032, GADD45A, INSR, MXI1, NOS3, PCK1, POMC, PPARGCIA, PRDX3, RBL2, SOD2 and TNFSF10 and/or wherein inferring the oxidative stress state of the FOXO transcription factor element is based on the expression levels of one or more, preferably all of the target genes of a FOXO transcription factor SOD2, BNIP3, MXI1 and PCK1 measured in the extracted sample of the medical subject;
inferring activity of a TGFbeta cellular signaling pathway in the sample based at least on expression levels of three or more, e.g. three, four five six seven, eight, nine, ten eleven, twelve or thirteen, target gene(s) of the TGFbeta pathway measured in the sample selected from the group comprising or consisting of: ANGPTL4, CDC42EP3, CDKNIA, CDKN2B, CTGF, GADD45A, GADD45B, HMGA2, ID1, IL11, SERPINE1, INPP5D, JUNB, MMP2, MMP9, NKX2-5, OVOL1, PDGFB, PTHLH, SGK1, SKIL, SMAD4, SMAD5, SMAD6, SMAD7, SNAI1, SNAI2, TIMP1, and VEGFA, preferably, from the group consisting of: ANGPTL4, CDC42EP3, CDKNIA, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, from the group consisting of: ANGPTL4, CDC42EP3, CDKNIA, CTGF, GADD45B, ID1, IL11, JUNB, SERPINE1, PDGFB, SKIL, SMAD7, SNAI2, and VEGFA, more preferably, from the group consisting of: ANGPTL4, CDC42EP3, ID1, IL11, JUNB, SERPINE1, SKIL, and SMAD7;
inferring activity of a NFkB cellular signaling pathway in the sample based at least on expression levels of three or more, e.g. three, four five six seven, eight, nine, ten eleven, twelve or thirteen, target gene(s) of the NFkB pathway measured in the sample selected from the group comprising or consisting of: BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKB IE, PTGS2, SELE, STAT5A, TNF, TNFAIP2, TNIP1, TRAF1, and VCAM1;
inferring activity of a JAK-STAT1/2 cellular signaling pathway in the sample based at least on expression levels of three or more, e.g. three, four five six seven, eight, nine, ten eleven, twelve or thirteen, target gene(s) of the JAK-STAT1/2 pathway measured in the sample selected from the group comprising or consisting of: BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFDIL, USP18, and ZNRF3, preferably, from the group consisting of: IRF1, IRF7, IRF8, IRF9, OAS1, PDCD1, ST13, STAT1, and USP18.
inferring activity of a JAK-STAT3 cellular signaling pathway in the sample based at least on expression levels of three or more, e.g. three, four five six seven, eight, nine, ten eleven, twelve or thirteen, target gene(s) of the JAK-STAT3 pathway measured in the sample selected from the group comprising or consisting of: AKT1, BCL2, BCL2L1, BIRC5, CCND1, CD274, CDKNIA, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM, and ZEB1, preferably, either from the group consisting of: BCL2L1, BIRC5, CCND1, CD274, FOS, HIF1A, HSP90AA1 , HSP90AB1 , MMP1 , and MYC, or from the group consisting of: BCL2L1, CD274, FOS, HSP90B1, HSPA1B, ICAM1, IFNG, JunB, PTGS2, STAT1, TNFRSF1B, and ZEB1;
inferring activity of a MAPK-AP1 cellular signaling pathway in the sample based at least on expression levels of three or more, e.g. three, four five six seven, eight, nine, ten eleven, twelve or thirteen, target gene(s) of the MAPK-AP1 pathway measured in the sample selected from the group comprising or consisting of: BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53, and VIM, preferably, from the group consisting of: CCND1, EGFR, EZR, GLRX, MMP1, MMP3, PLAU, PLAUR, SERPINE1, SNCG, and TIMP1;
inferring activity of a Notch cellular signaling pathway in the sample based at least on expression levels of three or more, e.g. three, four five six seven, eight, nine, ten eleven, twelve or thirteen, target gene(s) of the Notch pathway measured in the sample selected from the group comprising or consisting of: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more, for example, three, four, five, six or more, Notch target genes are selected from the group consisting of: DTX1, HES1, HES4, HES5, HEY2, MYC, NRARP, and PTCRA, and one or more, for example, two, three, four or more, Notch target genes are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC;
inferring activity of a PR cellular signaling pathway in the sample based at least on expression levels of three or more, e.g. three, four five six seven, eight, nine, ten eleven, twelve or thirteen, target gene(s) of the PR pathway measured in the sample selected from the group comprising or consisting of: [PR TARGET GENES]

Herein, a FOXO transcription factor (TF) element is defined to be a protein complex containing at least one of the FOXO TF family members, i.e., FOXO1, FOXO3A, FOXO4 and FOXO6, which is capable of binding to specific DNA sequences, thereby controlling transcription of target genes.

Herein, a Wnt transcription factor (TF) element is defined to be a protein complex containing at least one of the TCF/LEF TF family members, i.e., TCF1, TCF3, TCF4 or LEF1, preferably wherein the Wnt TF element comprises beta-catenin/TCF4, which is capable of binding to specific DNA sequences, thereby controlling transcription of target genes.

Herein, a HH transcription factor (TF) element is defined to be a protein complex containing at least one of the GLI TF family members, i.e., GLI1, GLI2 or GLI3 which is capable of binding to specific DNA sequences, thereby controlling transcription of target genes.

Herein, an AR transcription factor (TF) element is defined to be a protein complex containing at least one or preferably a dimer of nuclear Androgen receptor. Herein, an ER transcription factor (TF) element is defined to be a protein complex containing at least one or preferably a dimer of nuclear Estrogen receptor, preferably an ERalpha dimer.

Herein, the term "TGFbeta transcription factor element" or "TGFbeta TF element" or "TF element" when referring to the TGFbeta pathway is defined to be a protein complex containing at least one or, preferably, a dimer of the TGFbeta members (SMAD1 , SMAD2, SMAD3 , SMAD5 and SMAD8 with SMAD4) or a trimer (two proteins from SMAD1, SMAD2, SMAD3, SMAD5 and SMAD8 with SMAD4), which is capable of binding to specific DNA sequences, thereby controlling transcription of target genes. Preferably, the term refers to either a protein or protein complex transcriptional factor triggered by the binding of TGFbeta to its receptor or an intermediate downstream signaling agent between the binding of TGFbeta to its receptor and the final transcriptional factor protein or protein complex. For example, it is known that TGFbeta binds to an extracellular TGFbeta receptor that initiates an intracellular "SMAD" signaling pathway and that one or more SMAD proteins (receptor-regulated or R-SMADs (SMAD1, SMAD2, SMAD 3, SMAD5 and SMAD8) and SMAD4) participate in, and may form a hetero-complex which participates in, the TGFbeta transcription signaling cascade which controls expression.

Herein, an NFkB transcription factor (TF) element is defined to be a protein complex containing at least one or, preferably, a dimer of the NFkB members (NFKB 1 or p50/p105, NFKB2 or p52/p100, RELA or p65, REL, and RELB), which is capable of binding to specific DNA sequences, thereby controlling transcription of target genes. Herein, the term "Notch transcription factor element" or "Notch TF element" or "TF element" is defined to be a protein complex containing at least the intracellular domain of one of the Notch proteins (Notch1, Notch2, Notch3 and Notch4, with corresponding intracellular domains N1ICD, N2ICD, N3ICD and N4ICD), with a co-factor, such as the DNA-binding transcription factor CSL (CBF1/RBP-JK, SU(H) and LAG-1), which is capable of binding to specific DNA sequences, and preferably one co-activator protein from the mastermind- like (MAML) family (MAML1, MAML2 and MAML3), which is required to activate transcription, thereby controlling transcription of target genes. Preferably, the term refers to either a protein or protein complex transcriptional factor triggered by the cleavage of one of the Notch proteins (Notch1, Notch2, Notch3 and Notch4) resulting in a Notch intracellular domain (N1ICD, N2ICD, N3ICD and N4ICD). For example, it is known that DSL ligands (DLL1, DLL3, DLL4, Jagged1 and Jagged2) expressed on neighboring cells, bind to the extracellular domain of the Notch protein/receptor, initiating the intracellular Notch signaling pathway and that the Notch intracellular domain participates in the Notch signaling cascade which controls expression.

Herein, the term "JAK-STAT1/2 transcription factor element" or "JAK-STAT1/2 TF element" or "TF element" when referring to the JAK-STAT1/2 is defined to be a protein complex containing at least a STAT1-STAT2 heterodimer or a STAT1 homodimer, which is capable of binding to specific DNA sequences, preferably the ISRE (binding motif AGTTTC NTTCNC/T) or GAS (binding motif TTC/A NNG/TAA) response elements, respectively, thereby controlling transcription of target genes. Preferably, the term refers to either a protein or protein complex transcriptional factor that is formed by different stimuli such as IFNs triggered by the binding of the stimulating ligand to its receptor resulting in downstream signaling.

Herein, the term "JAK-STAT3 transcription factor element" or "JAK-STAT3 TF element" or "TF element" when referring to the JAK-STAT3 pathway is defined to be a protein complex containing at least a STAT3 homodimer, which is capable of binding to specific DNA sequences, preferably the response elements with binding motif CTGGGAA, thereby controlling transcription of target genes. Preferably, the term refers to either a protein or protein complex transcriptional factor triggered by the binding of STAT3 inducing ligands such as interleukin-6 (IL-6) and IL-6 family cytokines to its receptor or an intermediate downstream signaling agent between the binding the ligand to its receptor and the final transcriptional factor protein or protein complex.

Herein, the term "AP-1 transcription factor element" or "AP-1 TF element" or "TF element" when referring to the MAPK-AP1 pathway is defined to be a protein complex containing at least a member of the Jun (e.g. c-Jun, JunB and JunB) family and/or a member of the Fos (e.g. c-Fos, FosB, Fra-1 and Fra-2) family and/or a member of the ATF family and/or a member of the JDP family, forming e.g. Jun∼Jun or Jun∼Fos dimers, capable of binding to specific DNA sequences, preferably the response elements 12-0-Tetradecanoylphorbol-13-acetate (TPA) response element (TRE) with binding motif 5'-TGA G/C TCA-3' or cyclic AMP response element (CRE) with binding motif 5'-TGACGTCA-3', thereby controlling transcription of target genes. Preferably, the term refers to either a protein or protein complex transcriptional factor triggered by the binding of AP-1 inducing ligands, such as growth factors (e.g., EGF) and cytokines, to its receptor or an intermediate downstream signaling agent, or triggered by the presence of an AP-1 -activating mutation.

In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope thereof.

## Claims

1. A method for estimating heterogeneity of a tumour based on values for two or more genome mutation and/or gene expression related parameters for at least two spatially differentiated areas in a sample, the sample being a tissue sample of a tumour or a liquid sample obtained from a subject having the tumour, the method comprising the steps of:
- determining a sample heterogeneity score for the heterogeneity of the sample based on variabilities of each measured value for the at least two spatially differentiated areas;
- estimating the heterogeneity of the tumour by extrapolating the score for the heterogeneity of the sample to the tumour, thereby providing a tumour heterogeneity score.

2. A method in accordance with claim 1, wherein the step of determining the sample heterogeneity score comprises the step of performing a Principal Component Analysis, PCA, for converting the two or more genome mutation and/or gene expression related parameters into principle components, thereby reducing correlations between the two or more genome mutation and/or gene expression related parameters.

3. A method in accordance with claim 2, wherein the step of performing the PCA comprises:
- determining mean values for each of the two or more genome mutation and/or gene expression related parameters based on the measured values;
- subtracting each of the measured values by a mean value of its corresponding genome mutation and/or gene expression related parameter.

4. A method in accordance with any of the claims 2 and 3, wherein the step of determining the sample heterogeneity score comprises the steps of:
- computing standard deviations for each of the principle components and determining the sample heterogeneity score for the heterogeneity of the sample based on the computed standard deviations.

5. A method in accordance with any of the claims 2 - 4, wherein said method further comprises the step of:
- providing a base transformation matrix, M, constructed from samples from different areas of tumours of multiple subjects.

6. A method in accordance with any of the previous claims, wherein the method further comprises one or more steps selected from:
- deciding a treatment strategy for the subject based at least in part on the tumour heterogeneity score;
- predicting a treatment outcome for the subject based at least in part on the tumour heterogeneity score;
- predicting a survival probability for the subject based at least in part on the tumour heterogeneity score;
- predicting resistance to a therapy
- deciding on the efficacy of therapy administered prior t0 the heterogeneity analysis
- deciding on resistance to therapy administered prior to the heterogeneity analysis.

7. A method in accordance with any of the previous claims, wherein said gene expression related parameters are gene expression levels of three or more target genes each of one or more cellular signalling pathway selected from the group consisting of ER, AR, HH, PI3K-FOXO, WNT, TGFbeta, NFkB, JAK-STAT1/2, JAK-STAT3, Notch, PR and MAPK-AP1, preferably wherein said gene expression related parameters are cellular signalling pathway activities based on the three or more target genes expression levels for said cellular signalling pathway, more preferably wherein said cellular signalling pathway activity is selected from the group consisting of ER, AR, HH, PI3K-FOXO, WNT, TGFbeta, NFkB, JAK-STAT1/2, JAK-STAT3, Notch, PR and MAPK-AP1 cellular signalling pathway activity.

8. A method in accordance with any of the previous claims, wherein said step of estimating said sample heterogeneity score for the heterogeneity of the sample, comprises any of:
- multiplying each of said variabilities of each measured value for the at least two spatially differentiated areas, and
- summing each of said variabilities of each measured value for the at least two spatially differentiated areas.

9. A method in accordance with any of the previous claims, wherein said step of estimating the heterogeneity of the tumour comprises:
- setting an upper bound for the tumour heterogeneity score as the sample heterogeneity score.

10. An apparatus comprising a processor configured to perform a method in accordance with any one of the claims 1 to 9.

11. An apparatus in accordance with claim 10, wherein said processor is further arranged for:
- scanning said sample;
- identifying, in said sample, an area of interest;
- measuring, for said area of interest, value for the at least two spatially differentiated areas for two or more genome mutation and/or gene expression related parameters.

12. A non transitory storage medium storing instructions that are executable by a processor to perform a method in accordance with any one of the claims 1 to 10.

13. A computer program comprising program code means for causing a processor to perform a method in accordance with any of the claims 1 - 9.

14. A signal representing a tumour heterogeneity score that indicates the heterogeneity of a tumour, wherein the tumour heterogeneity score results from performing a method in accordance with any of the claims 1 - 10.

15. Use of the apparatus according to claim 10 or 11, the non-transitory storage medium according to claim 12, the computer program according to claim 13 and/or the signal according to claim 14 for diagnosing the subject, predicting a treatment outcome for the subject or predicting an optimal treatment strategy for the subject, wherein the diagnosing or predicting is based on the tumour heterogeneity score.
